# FASCICULE DE BREVET EUROPEEN

(11) **EP 3 727 305 B1**
(45) Date de publication et mention de la délivrance du brevet: **26.01.2022**
(21) Numéro de dépôt: 18833488.2
(22) Date de dépôt: 17.12.2018
(51) Int. Cl.: A61K 8/39, A61Q 1/04, A61K 8/81, A61K 8/86, A61K 8/06

(54) **EMULSION HUILE-DANS-EAU POUR LE MAQUILLAGE ET LE SOIN DES LEVRES**
ÖL-IN-WASSER-EMULSION ZUM SCHMINKEN UND ZUR PFLEGE DER LIPPEN
OIL-IN-WATER EMULSION FOR MAKING UP AND CARING FOR THE LIPS

(30) Priorité: 22.12.2017 FR 1763099
(43) Date de publication de la demande: 28.10.2020
(73) Titulaire: LVMH Recherche, 45800 Saint Jean de Braye (FR)
(72) Inventeur: DUVERT, Amandine, 45570 DAMPIERRE EN BURLY (FR); FABRIS, Marine, 45800 SAINT JEAN DE BRAYE (FR); PREVOT, Aline, 92200 Neuilly-sur-Seine (FR); DURTSCHI, Aurore, 45800 SAINT JEAN DE BRAYE (FR); KURFURST, Chantal, 45800 SAINT JEAN DE BRAYE (FR)
(74) Mandataire: Cabinet Beau de Loménie
(86) Numéro de dépôt international: PCT/FR2018/053337
(87) Numéro de publication internationale: WO 2019/122659

(56) Documents cités:
- EP-A1- 1 380 288
- EP-A1- 2 018 838
- CA-A1- 2 796 883
- US-A1- 2004 191 191
- DATABASE GNPD [Online] MINTEL; mai 2014 (2014-05), "Fluid Stick Lipstick", XP002782677, Database accession no. 2401385 cité dans la demande
- DATABASE GNPD [Online] MINTEL; mai 2018 (2018-05), "Lacquer Plump", XP002782678, Database accession no. 5674659
- "Fluid Stick Lipstick", GNPD, MINTEL, 1 May 2014 (2014-05-01), XP002782677,

## Description

L'invention concerne un produit pour le maquillage et le soin des lèvres sous la forme d'une émulsion huile-dans-eau. Cette émulsion contient l'association de deux huiles visqueuses en haute proportion, de pigments et d'un système dispersant en phase aqueuse.

### ART ANTERIEUR

Les produits pour lèvres brillants sont généralement anhydres et constitués d'huiles brillantes de haute viscosité et de polymère de tenue collant. Le film obtenu est alors lourd et épais.

Un moyen d'obtenir un film plus fin consiste à émulsionner ces huiles dans la phase interne d'une émulsion huile-dans-eau. Le film obtenu peut être plus fin, d'une part parce que l'émulsion glisse plus facilement sur les lèvres que la forme anhydre au moment de l'application et d'autre part, parce que l'épaisseur du film diminue par évaporation de l'eau sur les lèvres après application.

Pour obtenir une émulsion huile-dans-eau contenant des huiles brillantes, les difficultés à surmonter sont cependant nombreuses.

Tout d'abord, elles sont particulièrement difficiles à stabiliser lorsqu'on y introduit des pigments en quantité suffisante pour obtenir un bel effet couvrant et teinté. Lorsque la stabilisation est faible, les gouttelettes d'huiles peuvent être détectées visuellement par le consommateur, et donnent une perception négative d'inhomogénéité du produit. Il est alors nécessaire de le conditionner dans un flacon opaque. Or, la présentation d'un produit de maquillage des lèvres liquide dans un flacon transparent est fortement appréciée par les consommateurs qui sont ainsi en mesure de percevoir immédiatement la couleur qu'ils recherchent, et il est souhaitable que la couleur perçue à travers le flaconnage transparent soit fidèle à la couleur du film de produit déposé sur les lèvres.

Ensuite, les huiles de la phase interne d'une émulsion huile-dans-eau développent généralement un film de faible brillance après dépôt du produit sur les lèvres, sans que l'on sache expliquer pourquoi.

Enfin, les consommateurs apprécient les rouges à lèvres qui procurent une sensation de confort, ne dessèchent pas les lèvres et maintiennent donc le niveau d'hydratation initial des lèvres. Mais, ils apprécient davantage les produits apportant simultanément maquillage et soin des lèvres, le soin visant principalement la réhydratation des lèvres.

Une solution proposée pour conserver une bonne brillance du maquillage et des teintes intenses a consisté à introduire, dans une émulsion huile-dans-eau, des alkylcelluloses en dispersion dans l'eau. Certes, le film laissé sur les lèvres après l'application de ce genre de produit est brillant, mais l'émulsion n'est pas assez fine pour être présentée dans un conditionnement transparent. Par ailleurs, il est difficile de réaliser un maquillage uniforme en couleur et en épaisseur, et d'obtenir un contour précis des lèvres lors de l'application du produit. Enfin, ce type de produit ne peut pas servir de produit de soin réhydratant des lèvres. Un exemple d'une de ces formulations est décrit dans la demande FR 2 964 868.

Le besoin subsiste par conséquent de fournir un produit liquide pour le maquillage des lèvres en émulsion huile-dans-eau d'aspect homogène dans un conditionnement transparent, formant un film sur les lèvres fin, très brillant et très uniforme et dont les contours sont précis. Le besoin subsiste également de proposer un produit destiné à la fois au maquillage et au soin des lèvres et doté d'un fort pouvoir hydratant.

La composition de la présente invention a permis d'atteindre ces objectifs.

### DESCRIPTION DE L'INVENTION

Selon un premier aspect de l'invention, l'invention concerne un produit liquide de maquillage des lèvres en émulsion huile-dans-eau comprenant:
- de 25 à 45% d'un mélange d'eau et d'au moins un polyol,
- de 35 à 60% en masse, de préférence de 40 à 50% en masse, et de préférence encore de 45 à 50% en masse d'un mélange d'huiles ayant chacune un indice de réfraction supérieur ou égal à 1,460 à une température comprise allant de 20°C à 25°C,
- de 0,01 à 20% en masse d'une matière colorante,
   les pourcentages étant exprimés par rapport à la masse de l'émulsion,
   caractérisée en ce qu'elle comprend un premier éther de l'alcool stéarylique et d'un polyéthylène glycol comprenant de 2 à 5 motifs oxyéthylényle, un deuxième éther de l'alcool stéarylique et d'un polyéthylène glycol comprenant de 15 à 25 motifs oxyéthylényle, et un copolymère d'acrylamido-2-méthyl propane sulfonate et d'hydroxyéthyl acrylate.

On entend par matière colorante un composé ou un mélange de composés choisi parmi les pigments et les colorants.

Le mélange du premier éther, du deuxième éther et du copolymère peut remplir la fonction de mélange stabilisant et permet avantageusement d'émulsionner de 35% à 60% de phase grasse brillante, de stabiliser une dispersion lipophile de pigment et de stabiliser l'émulsion. La juste stabilisation de l'émulsion permet la coalescence des gouttelettes de la phase grasse lors de l'évaporation de l'eau sur les lèvres pour en révéler la brillance, en ne laissant aucun d'amas gélifiés sur les lèvres après le séchage du film.

Il est très surprenant d'obtenir une émulsion pigmentée, à la fois fluide et stable, contenant jusqu'à 50% en masse de phase interne, assurant une double performance de soin et de maquillage des lèvres. Un tel niveau de brillance pour un dépôt de produit sur les lèvres aussi fin n'avait jamais été obtenu auparavant.

L'invention décrit par ailleurs un produit liquide de maquillage des lèvres en émulsion huile-dans-eau comprenant:
- de 25 à 45% en masse, par exemple de 30 à 40% en masse, d'une phase aqueuse comprenant de l'eau et au moins un polyol,
- de 0,01 à 20% en masse d'une matière colorante,
- de 1 à 4% en masse d'au moins deux composés tensio-actifs non ioniques,
caractérisé en ce qu'elle contient en outre un copolymère de l'acrylamido-2-méthyl propane sulfonate, et de 35 à 60% en masse d'huiles dont 15% à 25% en masse d'un mélange de rosinate de méthyle hydrogéné et de polycyclopentadiène hydrogéné, les pourcentages étant exprimés par rapport à la masse du produit.

L'émulsion de l'invention permet d'obtenir un résultat maquillage de haute brillance, léger sur les lèvres, sans être collant. Cette émulsion contient une haute teneur en huiles pourtant réputées collantes et visqueuses par l'homme du métier de la cosmétique, ainsi qu'un système stabilisant particulier contenant deux tensio-actifs et un polymère.

Il est très surprenant de pouvoir incorporer et stabiliser une proportion aussi élevée d'huiles visqueuses dans la phase interne d'une émulsion huile-dans-eau, tout en conservant sa stabilité et tout en révélant la brillance des huiles dans le dépôt laissé sur les lèvres après application du produit et évaporation de l'eau. Sans être liés par aucune théorie, les inventeurs pensent que la combinaison d'ingrédients utilisée a permis de trouver un équilibre très pointu entre la stabilisation des gouttelettes d'huile dans l'émulsion pendant le stockage du produit, et l'aptitude de ces gouttelettes à coalescer au cours de l'évaporation de l'eau dans le film de produit qui a été déposé sur les lèvres. Ces deux propriétés sont a priori incompatibles dans la mesure où, plus les gouttelettes sont stabilisées, plus leur coalescence devrait être difficile. Cette propriété inattendue de l'émulsion de la présente invention, permet ainsi d'émulsionner des huiles brillantes réputées difficiles à émulsionner de façon stable, et d'obtenir sur les lèvres un film d'huiles continu dont l'état de surface régulier et lissé permet de conserver la brillance intrinsèque des matières premières et d'obtenir un effet laqué.

C'est pourquoi, bien que les huiles entrant dans la composition de l'émulsion soient connues indépendamment les unes des autres dans le domaine de la formulation des produits pour lèvres brillants anhydres, et bien que les agents stabilisants introduits dans l'émulsion de l'invention aient déjà été utilisés pour stabiliser des émulsions huile-dans-eau ne contenant pas des huiles brillantes, mais des huiles très légères faciles à émulsionner, l'effet de la combinaison de ces matières premières ne pouvait en aucun cas être prévisible, ce d'autant plus que les performances de brillance et d'hydratation du rouge à lèvres liquide en émulsion de l'invention n'ont jamais été atteintes jusqu'à présent.

Par ailleurs, les solutions qui ont été proposées dans l'art antérieur pour améliorer la brillance d'un rouge à lèvres en émulsion huile-dans-eau ne reposent pas sur la structuration de la phase interne de l'émulsion, mais sur l'utilisation de polymères filmogènes dans la phase aqueuse. L'invention propose donc une voie de formulation très différente pour atteindre des objectifs de brillance et d'hydratation plus élevés.

La stabilité de l'émulsion de l'invention permet de la conditionner dans des flacons transparents ce qui rend le produit beaucoup plus attractif : cela permet notamment à l'utilisateur de bien visualiser la teinte.

D'autres aspects de l'invention portent sur i) un procédé de maquillage ou de soin des lèvres qui consiste à appliquer sur les lèvres une des émulsions décrites précédemment, ii) un procédé de fabrication d'une des émulsions décrites précédemment, et iii) un flacon transparent doté d'un applicateur contenant une des émulsions décrites précédemment.

Dans la suite du texte les pourcentages sont exprimés en masse par rapport à la masse de l'émulsion de l'invention, sauf mention explicite contraire.

Dans la présente demande l'expression « de...à ...» vise à comprendre les bornes inférieure et supérieure de la plage de valeurs, tandis que l'expression « entre ... et... » exclut les bornes de la plage de valeurs. La divulgation d'une plage de valeurs excluant ses bornes vaut divulgation de la plage de valeurs équivalente incluant les bornes, et *vice versa.*

Les huiles entrant dans la composition de l'émulsion de l'invention représente de préférence de 35% à 60% en masse, de préférence encore de 40 à 50% en masse, et plus préférentiellement de 45 à 50% en masse. Ces huiles sont qualifiées par un homme du métier d'huiles lourdes, visqueuses ou brillantes selon les cas.

Ces huiles peuvent par exemple avoir un indice de réfraction, mesuré à une température allant de 20°C à 25°C, qui est supérieur ou égal à 1,460 et de préférence allant de 1,460 à 1,580.

L'indice de réfraction de l'huile peut être mesuré par toute méthode connue de l'homme du métier de la cosmétique.

Une méthode permettant de mesurer l'indice de réfraction d'une huile utilise un réfractomètre de la société Anton Paar modèle Abbemat^{®} 300/500. La température de mesure est réglée à 20°C ou 25°C. Un millilitre de l'huile dont on veut mesurer l'indice de réfraction est déposé à la pipette sur le prisme propre et la mesure est effectuée automatiquement par l'appareil.

L'homme du métier de la cosmétique peut également se référer aux données techniques fournies par le fabricant de l'huile.

Des exemples de telles huiles sont le phénylpropyl diméthylsiloxysilicate de marque Silshine^{®}151 (n=1,509), le POLYGLYCERYL-10 NONAISOSTEARATE de référence commerciale SFACE^{®} IS 1009 P (n=1,492), le polybutène de marque Polybutene^{®} M 2000 (n=1,492), le TRIMETHYL PENTAPHENYL TRISILOXANE de marque DC PH^{®} 1555 (n=1,579), le 1,2,4-BENZENETRICARBOXYLIC ACID, BRANCHED TRIDECYL ISODECYL ESTERS de référence commerciale Liponate^{®} TDTM (n=1,483), le dilinoléate de dilinoléyle de marque Lusplan^{®} DD DA7 (n=1,482), le copolymère de polyglycéryle-2 isostéarate et de dimère dilinoléate de référence commerciale Hailuscent^{®} ISDA (n=1,476), le DIPHENYL DIMETHICONE de marque KF^{®} 54 HV (n=1,495-1,505) et le TRIMETHYLOLPROPANE TRIISOSTEARATE de référence commerciale Salacos^{®}6318 V (n=1,466). Dans ce paragraphe, les indices de réfraction ont été mesurés selon la méthode décrite plus haut à une température de 20°C.

L'huile peut être également choisie parmi le malate de diisostéaryle, l'octyldodécanol, les polybutènes, les copolymères de vinylpyrrolidone, les polyisobutènes hydrogénés, les polydécènes, le tri-isostéarate de poylglycérol-2, le tridécyl trimellitate, le ditriméthylolpropane isostéarate/sébaçate, le dipentaérythrityl tri-polyhydroxystéarate, dipentaérythrityl pentaisostéarate, le pentaérythrityl tétraisostéarate, le tétra-isostéarate de poylglycérol-2 et l'huile de ricin. L'indice de réfraction de ces huiles fait partie des caractéristiques techniques fournies par le fabricant.

On préfère utiliser comme huile brillante, un ester de rosine hydrogéné, un polycyclopentadiène hydrogéné, un polyisobutène hydrogéné ou un des mélanges de ces trois huiles, notamment un mélange d'ester de rosine hydrogéné et de polycyclopentadiène hydrogéné, ou un mélange d'ester de rosine hydrogéné, de polycyclopentadiène hydrogéné et de polyisobutène hydrogéné.

La rosine (encore appelé acide rosinique) est un acide aromatique comprenant un motif glucopyranoside. Il est extrait de la colophane, laquelle est obtenue après distillation et séchage d'un exsudat récolté à partir des arbres résineux tels que les pins. L'ester de rosine est de préférence le rosinate de méthyle hydrogéné (de dénomination INCI METHYL HYDROGENATED ROSINATE) tel que celui commercialisé sous le nom FLORALYN^{®} par la société LASERSON. L'ester de rosine peut représenter entre 10 et 15% ne masse. Son indice de réfraction est égal à 1,515.

Le polycyclopentadiène hydrogéné est par exemple introduit dans l'émulsion à une teneur allant de 2 à 5% en masse, notamment en mélange avec une autre huile. Un tel mélange peut correspondre au produit commercial KOGOGUARD^{®} 5400 CCT fabriqué par la société KOBO. Son indice de réfraction à 20°C peut être de 1,503 et sa viscosité mesurée à 25°C peut être de 2849 cPs.

Enfin, le polyisobutène hydrogéné peut être celui de référence commerciale Parleam^{®}HV (n=1,497) de la société NOF CORPORATION, et être présent à hauteur de 6 à 10% en masse.

L'émulsion peut contenir d'autres huiles que les huiles décrites précédemment, mais de préférence en une quantité allant de 0 à 15%, de préférence inférieure à 10%. Ces huiles peuvent entrer dans la composition des matières premières utilisées pour la fabrication de l'émulsion. Elles peuvent également servir de moyen de dispersion des pigments utilisés pour la coloration des lèvres. Un triglycéride caprylique/caprique correspond à une de ces huiles.

Dans un mode de réalisation particulier, l'émulsion comprend le mélange d'un premier éther de l'alcool stéarylique et d'un polyéthylène glycol comprenant de 2 à 5 motifs oxyéthylényle, d'un deuxième éther de l'alcool stéarylique et d'un polyéthylène glycol comprenant de 15 à 25 motifs oxyéthylényle, et d'un copolymère de l'acrylamido-2-méthyl propane sulfonate.

Le premier éther peut avoir pour dénomination INCI Steareth-2 ou dénomination commerciale Brij^{™} S2, et le deuxième éther pour dénomination INCI Steareth-21 ou dénomination commerciale Brij^{™}S721.

Le copolymère de l'acrylamido-2-méthyl propane sulfonate est par exemple un copolymère d'acrylamido-2-méthyl propane sulfonate et d'hydroxyéthyl acrylate. Il représente de préférence de 0,2% à 1,0% en masse, par exemple de 0,6 à 0,9% en masse de la masse de l'émulsion. Dans un procédé de préparation de l'émulsion de l'invention, le copolymère peut être ajouté aux autres ingrédients de l'émulsion après avoir été prédispersé dans de l'eau. Une prédispersion du copolymère prête à l'emploi est commercialisée sous la marque Simulgel^{®} NS par la société SEPPIC.

L'émulsion de l'invention peut contenir d'autres composés tensio-actifs ou des agents coémulsionnants, comme le myristate myreth-3 (par exemple de la marque Lanol^{®}14 M), le polysorbate 60 et le sorbitan isotearate. Ces agents émulsionnants peuvent notamment être utilisés pour préparer une prédispersion du copolymère et faciliter son incorporation dans l'émulsion.

L'invention décrit également une émulsion de l'invention contenant un mélange d'au moins un polymère présent dans la phase aqueuse, et d'au moins deux tensio-actifs non ioniques, dont un tensio-actif non ionique de HLB inférieur à 8 et un tensio-actif non ionique de HLB supérieur à 8. Ce mélange peut avantageusement stabiliser la dispersion des huiles et des pigments.

L'émulsion de l'invention comprend de préférence moins de 0,2% en poids, de préférence moins de 0,15% en poids d'un des composés mentionnés dans la liste suivante: l'hydroxyéthylcellulose (comme celles de la gamme Natrosol^{®}), les polyacrylates réticulés (comme les produits de dénomination INCI POLYACRYLATE CROSSPOLYMER-6), les gommes de xanthane (par exemple les gommes de marque Rhodicare XC et Rhodicare T), les carraghénanes, les hydroxyméthylcellulloses, les éthylcelluloses en dispersion, le pullulane, l'agar, un polysaccharide produit par la bactérie *Alcaligenes* (par exemple le produit de marque Alcasealan^{®}), les gels de polyuréthane (de la gamme AdekaNol^{®} notamment).

L'émulsion de l'invention est avantageusement dépourvue de l'un des gélifiants précités.

Il a en effet été démontré que des émulsions contenant ces composés ne sont pas stables : on observe un déphasage des huiles et de l'eau, la sédimentation de particules solides, une consistance du produit trop fluide et/ou la formation d'amas de produit sur les lèvres, une fois le produit déposé.

L'émulsion peut contenir de 25 à 45% en poids, par exemple de 30 à 40% en poids d'eau.

Outre de l'eau, la phase aqueuse de l'émulsion peut contenir le copolymère d'acrylamido-2-méthyl propane sulfonate et d'hydroxyéthyl acrylate décrit précédemment, des ajusteurs de pH (comme par exemple un citrate et/ou de l'acide citrique), de l'éthanol, au moins un polyol, tel que le glycérol ou le butylène glycol, et des édulcorants comme le stévioside de référence commerciale Rebaten.

Ainsi, l'émulsion de l'invention peut comprendre de 3% à 15% en poids de polyol(s), en particulier de 5% à 10% en poids de polyol(s) par rapport au poids de l'émulsion.

L'émulsion de l'invention présente l'originalité d'être très fluide en comparaison des produits liquides existant pour le maquillage des lèvres

La viscosité de l'émulsion à 25°C et pression atmosphérique est de préférence comprise entre 1.000 et 10.000 mPa.s, de préférence encore comprise entre 1.000 et 5.000 mPa.s.

Cette viscosité peut être mesurée avec un viscosimètre Rhéolab QC (Anton Paar) doté du logiciel Rheoplus en utilisant un mobile 4 Pales ST22 - 4V (vitesse de rotation 100 tr/min et temps de mesure 3 min).

Préalablement à la mesure, on place l'émulsion de l'invention dans un pot de 120 ml (Réf : 102171001, Kola Rond VT3 M120 Blanc Pharm) dans une étuve à 25°C pendant 12 heures minimum. Une fois le mobile plongé dans le pot, le niveau de composition doit atteindre le col du pot.

On vérifie que le mobile est bien choisi en mesurant le pourcentage de déviation des mesures qui sont effectuées toutes les 6 secondes. La valeur de la viscosité de l'émulsion est égale, selon ce protocole, à la moyenne des quinze dernières mesures effectuées par l'appareil pendant le temps de mesure ci-dessus indiqué.

L'émulsion de l'invention peut contenir une ou plusieurs molécules et/ou un ou plusieurs extraits végétaux présentant des propriétés hydratantes, tels que les glycols mentionnés précédemment, des polyols naturels, de l'aloe vera, un hyaluronate de sodium, un saccharide isomerate (le Pentavitine^{®} par exemple), l'aloe vera, et tout autre actif hydratant connu de l'homme de l'art.

Parmi les pigments minéraux, on peut citer, à titre d'exemples le dioxyde de titane, éventuellement traité en surface; les oxydes de fer noir, jaune, rouge et brun; et le violet de manganèse.

Parmi les pigments minéraux, on peut citer, à titre d'exemples le dioxyde de titane; les oxydes de fer noir, jaune, rouge et brun et le violet de manganèse.

Parmi les pigments organiques, on peut citer, par exemple, les pigments D & C Red n° 27 ; D & C Red n° 22 ; D & C Red n° 21 ; D & C Red n° 28 ; D & C orange n° 4 ; D & C Red n° 33 ; D & C Red n° 7 ; D & C Red n° 6 ; D & C Yellow n° 5 ; D & C Red n° 36 ; D & C Yellow n° 6 ; D & C Red n° 30 ; D &C Blue 1 et les laques à base de carmin de cochenille.

Parmi les colorants, on pourra citer Yellow 5, Yellow 6, Blue 1, Green 5, Green 3, Green 6, Orange 4, Red 4, Red 21, Red 22, Red 27, Red 28, Red 33, Red 40, le carmin de cochenille (Cl 15850, Cl 75470). Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou.

Les pigments nacrés peuvent être choisis notamment parmi les pigments nacrés blancs, tels que le mica recouvert d'oxyde de titane, l'oxychlorure de bismuth ; et les pigments nacrés colorés, tels que le mica titane avec des oxydes de fer, mica titane avec un pigment organique du type précité, ainsi que les pigments à base d'oxychlorure de bismuth. Ces pigments peuvent avoir les dénominations INCI suivantes : CALCIUM SODIUM BOROSILICATE, TIN OXIDE, SYNTHETlC FLUORPHLOGOPITE, SILICA, ALUMINA, ALUMINUM HYDROXIDE, MICA et CALCIUM ALUMINIUM BOROSILICATE.

L'émulsion de l'invention contient par exemple de 0,01 à 20% en masse de matière colorante par rapport à la masse de l'émulsion. Les nacres peuvent ainsi représenter de 0 à 15% en masse, les pigments organiques et inorganiques de 0 à 3% et les colorants de 0,01 à 1% en masse, de la masse de l'émulsion.

Outre les ingrédients décrits précédemment, l'émulsion de l'invention comprend au moins un excipient cosmétiquement ou dermatologiquement acceptable qui peut être choisi parmi des parfums, des électrolytes, des édulcorants pour masquer l'amertume de certains composés de l'émulsion lorsque celle-ci est appliquée sur les lèvres, des ajusteurs de pH et des conservateurs, des filtres solaires ou encore des anti-oxydants.

L'invention concerne également un procédé de soin ou de maquillage des lèvres qui consiste à appliquer sur les lèvres un produit tel que décrit précédemment. Toutes les caractéristiques qui ont été décrites en rapport avec ces produits s'appliquent au procédé de maquillage de l'invention.

L'émulsion de l'invention selon un des aspects décrits précédemment, est un produit de soin ou de maquillage des lèvres qui procure un rendu très brillant, et qui n'est pas adapté à d'autres utilisations telles que le soin ou le maquillage de la peau pour lequel la brillance est un défaut rédhibitoire : c'est le cas notamment des produits de maquillage ou de soin en émulsion huile-dans-eau de type lotion, sérum, crème ou fond de teint.

L'émulsion comprend de préférence moins de 1% en poids, de préférence moins de 0,5%, voire même être dépourvue de cires dont le point de fusion est supérieur à 70°C, car leur cristallisation crée de la matité, ce que l'on veut précisément éviter.

L'invention concerne un flacon, de préférence transparent, doté d'un moyen d'application contenant le produit à lèvres décrit précédemment. Le produit selon l'invention est avantageusement conditionnée dans un flacon (ou bouillotte) transparent doté d'un moyen d'application et d'un capot (ou bouchon). Le moyen d'application peut être un pinceau ou une mousse alvéolée, et être avantageusement fixé au capot. Le pinceau est de préférence plat et son extrémité peut être droite ou arrondie. Le flacon peut avoir une forme de cylindre, de cube ou de parallélépipède. L'applicateur peut avoir différentes formes - cylindrique, oblongue ou plate par exemple - et être éventuellement biseauté pour améliorer la précision de l'application de l'émulsion sur les lèvres.

L'invention concerne enfin un procédé de préparation d'une des émulsions décrites précédemment.

Un mode de réalisation particulier de ce procédé comprend au moins trois étapes. Dans une première étape on mélange les ingrédients de la phase grasse et on y disperse un broyé de pigments préparé au préalable. Dans une deuxième étape, on solubilise tous les ingrédients de la phase aqueuse dans de l'eau. Enfin, dans une troisième étape, on mélange les deux phases préalablement chauffées à une température de l'ordre de 70°C à 80°C. Des actifs et des parfums peuvent être ajoutés à température ambiante.

L'invention est illustrée plus en détail par les exemples suivants.

### Exemple 1: Invention

On prépare un rouge à lèvre liquide dont la formule est présentée dans le Tableau 1. Les pourcentages sont en poids de la composition.

**Tableau 1**

| Phase | Dénomination INCI en majuscules ou fonction en minuscule | % |
|---|---|---|
| A1 | METHYL HYDROGENATED ROSINATE | 10 |
| A1 | HYDROGENATED POLYISOBUTENE | 9 |
| A1 | HYDROGENATED POLYCYCLOPENTADIENE | 4 |
| A1 | BIS-BEHENYL/ISOSTEARYL/PHYTOSTERYL DIMER DILINOLEYL DIMER DILINOLEATE | 6 |
| A1 | CAPRILIC/CAPRIC TRIGLYCERIDES | 4 |
| A1 | STEARETH-21 | 2.4 |
| A1 | MYRETH-3 MYRISTATE | 0.5 |
| A1 | STEARETH-2 | 0.5 |
| A2 | POLYGLYCERYL-2 TRIISOSTEARATE | 9.5 |
| A2 | Pigments | 3 |
| B1 | AQUA | Qsp 100 |
| B1 | GLYCERIN | 5 |
| B1 | Conservateurs | Qs |
| B1 | Colorants | 0.12 |
| B2 | HYDROXYETHYL ACRYLATE/SODIUM ACRYLOYLDIMETHYL TAURATE COPOLYMER | 0.7 |
| B2 | POLYSORBATE 60 | 0.1 |
| B2 | SORBITAN ISOSTEARATE | 0.03 |
| B2 | SQUALANE | 0.5 |
| B3 | Actif | 1.6 |
| C | ALCOHOL | 3 |
| C | Parfum | Qs |

### Procédé de Préparation:

Phase A : on disperse A2 dans A1 pendant 5 min dans un homogénéiseur de marque Turrax^{®} à une vitesse d'agitation de 10000 tr/min, et l'on chauffe la phase A à 75°C.

Phase B : on solubilise les colorants dans l'eau sous agitation émulseur de marque Rayneri pendant 20 min, puis l'on chauffe la phase B à 75°C.

On émulsionne ensuite la phase B dans la phase A avec un émulseur de marque Rayneri^{®} pendant 10 min à une vitesse d'agitation de 2000 tr/min, puis on laisse refroidir en maintenant l'agitation jusqu'à 40°C, pour ensuite ajouter les phases B2, B3, puis B4. On laisse refroidir ce mélange à 35°C, puis on ajoute C.

### • Mesure de la viscosité

La viscosité de l'émulsion de l'exemple 1 selon l'invention a été mesurée avec un viscosimètre Rhéolab^{®} QC (Anton Paar) doté du logiciel Rheoplus^{®}, avec le mobile Ailette tournant à 50 tour/min pendant 3 minutes.

Préalablement à la mesure, la composition est versée dans un pot de 120 ml (Réf : 102171001, Kola Rond^{®} VT3 M120 Blanc Pharm) puis l'ensemble est placée dans une étuve à 25°C pendant 12 heures minimum. Une fois le mobile plongé dans le pot, le niveau de composition atteint le col du pot.

La valeur de la viscosité de l'émulsion est égale à la moyenne des quinze dernières mesures effectuées par l'appareil pendant le temps de mesure indiqué ci-dessus.

Résultat : la viscosité de l'exemple 1 de l'invention était égale à 2560 mPa.s.

### • Homogénéité

On a observé au microscope, à un grossissement de 200 microns, l'émulsion préparée précédemment et un produit commercial sous la forme d'une émulsion eau-dans-huile (Fiche Mintel N° N°2401385).

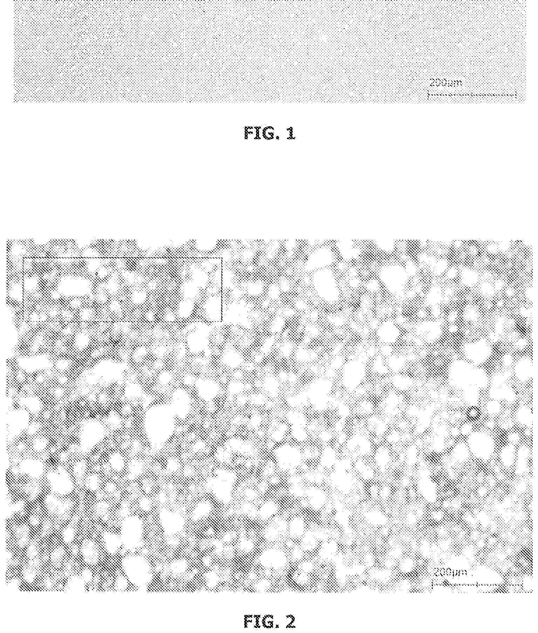

Les Figures 1 et 2 présentent les clichés de microscopie respectivement pour le produit de l'invention et le produit de l'art antérieur.

### • Etude d'obiectivation cutanée : mesure du pouvoir hydratant

Le pouvoir hydratant de l'Exemple 1 de l'invention a été mesuré au moyen d'un cornéomètre CM 825 (Courage et Khazaka) sur 11 volontaires caucasiens, en réalisant une application sur l'avant-bras (T= 0).

Deux zones de peau de 25 centimètres carrés sont choisies selon une distribution aléatoire sur la face intérieure de l'avant-bras de chaque volontaire. L'émulsion est appliquée sur l'une des deux zones déterminées à raison de 2 mg/cm² de façon « in use », la deuxième zone non traitée sert de témoin.

On définit l'effet hydratant de l'émulsion au temps T=X heures, comme étant égal au pourcentage d'augmentation entre la capacitance mesurée au temps T=X heures sur une zone de peau de l'avant-bras non traitée et la capacitance mesurée au temps T=X heures sur une zone de peau de l'avant-bras sur laquelle on a appliqué l'émulsion de l'Exemple 1 selon l'invention après retrait du film résiduel.

On a suivi le même protocole pour mesurer le pouvoir hydratant d'un produit commercial sous la forme d'une émulsion eau-dans-huile (Fiche Mintel N° N°2401385).

Résultats :
L'émulsion de l'invention présentent un pouvoir hydratant (+84% à T=16 heures et +78% à T=24 heures) plus élevé que celui du produit de l'art antérieur (+16% à T=6 heures). Cet effet se prolonge dans le temps.

### • Evaluation de l'effet repulpant

Le produit de l'exemple 1 de l'invention a été évalué sur un panel de 20 femmes volontaires âgées de 18 à 44 ans (moyenne d'âge de 35.0 ans).

Le produit a été appliqué une seule fois sur les lèvres et deux observations sont réalisées :
- les lèvres sont prises en photo avant et après application du produit,
- un expert dermatologue examine les lèvres avant et après application du produit.

L'analyse d'image a permis d'établir que le produit de l'exemple 1 procure un effet repulpant immédiat. La moyenne du pourcentage de variation de l'aire sous la courbe avant et après application du produit était de +25%. L'angle muco-cutané a augmenté de 3% et l'angle de courbure a diminué de 9%.

L'évaluation clinique l'a confirmé. Le pourcentage de variation de l'effet repulpant déterminé par le dermatologue était de +29% pour l'ensemble du panel.

### • Analyse sensorielle

Un panel des 14 personnes entraînées à l'analyse sensorielle des rouges à lèvres liquides est constitué.

Chaque membre du panel attribue une note allant de 0 à 10 pour chacun des descripteurs mentionnés dans le tableau 2 ci-dessous, et évalue séparément le rouge à lèvres de l'Exemple 1 et le produit de l'art antérieur (Fiche Mintel N° N°2401385, Teinte numéro 9). La couvrance correspond à l'uniformité de la couleur après un seul passage du produit.

Les résultats qui sont significatifs (alpha inférieur ou égal à 10%) sont présentés dans le Tableau 2.

**Tableau 2**

| | **Note sur 10** | |
|---|---|---|
| **Descripteur** | **Exemple 1** | **Rouge à lèvres liquide de l'art antérieur** |
| Couvrance | 7.6 | 6.8 |
| Contour précis | 7.6 | 7.0 |
| Film Uniforme en couleur et en épaisseur | 7.2 | 6.2 |
| Film Brillant | 7.4 | 6.7 |
| Fidélité de la couleur | 7.9 | 7.6 |
| Effet laqué de brillance très uniforme | 6.1 | 5.7 |
| Lèvres Pulpeuses | 7.1 | 6.5 |

### • Auto-évaluations

Le produit de l'exemple 1 de l'invention a été évalué par un panel de 32 femmes volontaires de type caucasien âgées de 20 à 65 ans (moyenne d'âge de 46.0 ans) qui ne sont pas entraînées à l'évaluation de produits cosmétiques. Chaque femme applique le produit sur les lèvres deux fois par jour pendant quatre semaines consécutives.

Chaque femme du panel a évalué elle-même les effets et les propriétés du produit en répondant à un questionnaire à l'issue de la période de test. Les volontaires répondent à un questionnaire en notant : « d'accord », « plutôt d'accord », « plutôt pas d'accord », « pas du tout d'accord ». Pour chaque item, le pourcentage de satisfaction (nombre de réponse « d'accord » et « plutôt d'accord ») est calculé.

Les résultats obtenus qui étaient statistiquement significatifs sont reportés dans le tableau 3 ci-dessous.

**Tableau 3**

| **Propriété évaluée** | **Pourcentage de satisfaction** |
|---|---|
| La texture est fraîche, légère et extra-fine | 84 |
| Le produit laisse un film fin et léger sur les lèvres | 81 |
| Le produit laisse un film non gras et non collant | 84 |
| La brillance est laquée | 94 |
| Un film brillant lissant se forme au-dessus de la couleur | 88 |
| La couvrance est satisfaisante | 84 |
| Les lèvres sont lissées | 91 |

Les consommateurs non avertis ont jugé le résultat maquillage obtenu avec l'émulsion de l'Exemple 1 selon l'invention très brillant et léger. Le film lisse les lèvres à tel point que le niveau de brillance atteint celui d'une laque.

Les consommateurs ont relevé des qualités identiques à celles qui ont été mise en évidence par le panel de personnes entraînées à l'évaluation de rouge à lèvres liquide, ce qui confirme la significativité des résultats.

### Exemple 2 comparatif :

On a préparé une composition non conforme à l'invention et dont la liste des ingrédients est reproduite dans le Tableau 4.

Cette composition contient 29,2% d'un mélange d'eau et de glycérine, 48,5% d'un mélange d'huiles brillantes, 3,1% de matière colorante et deux éthers de l'alcool stéarylique (Steareth-2 et Steareth-21). Elle est dépourvue du copolymère d'acrylamido-2-méthyl propane sulfonate et d'hydroxyéthyl acrylate, qui a été substitué par une gomme de xanthane.

**Tableau 4**

| Phase | Dénomination INCI en majuscules ou nom chimique en minuscule | % |
|---|---|---|
| A1 | PHENYLPROPYLDIMETHYLSILOXYSILICATE | 23 |
| A1 | HYDROGENATED POLYISOBUTENE | 9 |
| A1 | HYDROGENATED POLYCYCLOPENTADIENE | 4 |
| A1 | BIS-BE H E NYL/ISOSTEARYL/PHYTOSTE RYL DIMER DILINOLEYL DIMER DILINOLEATE | 4 |
| A1 | CAPRILIC/CAPRIC TRIGLYCERIDES | 4 |
| A1 | STEARETH-21 | 2.2 |
| A1 | MYRETH-3 MYRISTATE | 2 |
| A1 | STEARETH-2 | 0.7 |
| A2 | POLYGLYCERYL-2 TRIISOSTEARATE | 9.5 |
| A2 | Pigments | 3 |
| B1 | AQUA | Qsp 100 |
| B1 | GLYCERIN | 5 |
| B1 | Conservateurs | 0.9 |
| B1 | Colorants | 0.1 |
| B2 | XANTHANE GUM | 0.4 |
| B3 | Actifs | 1.6 |
| C | ALCOHOL | 3.4 |
| C | Parfum | 3 |

L'émulsion est préparée selon le procédé de l'exemple 1. Elle est instable : on observe une sédimentation des pigments et un déphasage entre la phase aqueuse et la phase grasse.

### Exemple 3 comparatif :

On a préparé une composition non conforme à l'invention et dont la liste des ingrédients est reproduite dans le Tableau 5.

Cette composition contient un mélange d'eau et de glycérine, 35,5% d'un mélange d'huiles brillantes, s matières colorantes. Les deux éthers de l'alcool stéarylique (Steareth-2 et Steareth-21) présents dans l'Exemple 1 ont été remplacés par le Glyceryl Stearate SE et le Sodium Stearoyl Glutamate. Elle est également dépourvue du copolymère d'acrylamido-2-méthyl propane sulfonate et d'hydroxyéthyl acrylate, qui a été substitué par une gomme de xanthane.

**Tableau 5**

| Phase | Dénomination INCI en majuscules ou fonction minuscule | % |
|---|---|---|
| A1 | METHYL HYDROGENATED ROSINATE | 10 |
| A1 | HYDROGENATED POLYISOBUTENE | 9 |
| A1 | HYDROGENATED POLYCYCLOPENTADIENE | 4 |
| A1 | BIS-BEHENYL/ISOSTEARYL/PHYTOSTERYL DIMER DILINOLEYL DIMER DILINOLEATE | 3 |
| A1 | CAPRILIC/CAPRIC TRIGLYCERIDES | 4 |
| A1 | GLYCERYL STEARATE SE | 5 |
| A1 | SODIUM STEAROYL GLUTAMATE | 2 |
| A2 | POLYGLYCERYL-2 TRIISOSTEARATE | 9.5 |
| A2 | Pigments | 3 |
| B1 | AQUA | Qsp 100 |
| B1 | GLYCERIN | 5 |
| B1 | Conservateurs | Qs |
| B1 | Colorants | 0.1 |
| B2 | XANTHANE GUM | 0.4 |
| B3 | Actifs | 1.6 |
| C | ALCOHOL | 3 |
| C | Parfum | Qs |

L'émulsion est préparée selon le procédé de l'exemple 1. Elle est instable : on observe une sédimentation des pigments et un déphasage entre la phase aqueuse et la phase grasse.

## Revendications

1. Produit liquide de maquillage et de soin des lèvres en émulsion huile-dans-eau comprenant:
- de 25 à 45% d'un mélange d'eau et d'au moins un polyol,
- de 35 à 60% en masse d'un mélange d'huiles ayant chacune un indice de réfraction, mesuré à une température allant de 20°C à 25°C, qui est supérieur ou égal à 1,460,
- de 0,01 à 20% en masse d'une matière colorante,
les pourcentages étant exprimés par rapport à la masse de l'émulsion,
**caractérisée en ce qu'**elle comprend un premier éther de l'alcool stéarylique et d'un polyéthylène glycol comprenant de 2 à 5 motifs oxyéthylényle, un deuxième éther de l'alcool stéarylique et d'un polyéthylène glycol comprenant de 15 à 25 motifs oxyéthylényle, et un copolymère d'acrylamido-2-méthyl propane sulfonate.

2. Produit selon la revendication 1, **caractérisé en ce que** les huiles comprennent le rosinate de méthyle hydrogéné et le polycylcopentadiène hydrogéné.

3. Produit selon la revendication 2, **caractérisé en ce que** le rosinate de méthyle hydrogéné représente entre 10 et 15% en masse de la masse du produit.

4. Produit selon l'une des revendications 2 et 3, **caractérisé en ce que** le polycyclopentadiène hydrogéné représente entre 2 et 5% en masse de la masse du produit.

5. Produit selon l'une des revendications précédentes, **caractérisé en ce que** le premier éther a pour dénomination INCI STEARETH-2 et le deuxième éther a pour dénomination INCI STEARETH-21.

6. Produit selon l'une des revendications précédentes, **caractérisé en ce que** le copolymère d'acrylamido-2-méthyl propane sulfonate et d'hydroxyéthyl acrylate représente entre 0,2 et 1,0 % en masse de la masse du produit.

7. Procédé de maquillage des lèvres qui consiste à appliquer sur les lèvres un produit selon l'une des revendications 1 à 6.

8. Flacon doté d'un moyen d'application et d'un capot, lequel flacon est transparent et contient un produit selon l'une des revendications 1 à 6, ledit moyen d'application étant un pinceau ou une mousse alvéolée.

## Patentansprüche

1. Flüssiges Schmink- und Lippenpflegeprodukt als Öl-in-Wasser-Emulsion, umfassend:
- 25 bis 45 % einer Mischung von Wasser und zumindest einem Polyol,
- 35 bis 60 Massen-% einer Mischung von Ölen, die jeweils einen Brechungsindex, gemessen bei einer von 20 °C bis 25 °C reichenden Temperatur, aufweisen, der höher oder gleich 1,460 ist,
- 0,01 bis 20 Massen-% eines Farbstoffs,
wobei die Prozentsätze bezogen auf die Masse der Emulsion ausgedrückt sind,
**dadurch gekennzeichnet, dass** es einen ersten Ether eines Stearylalkohols und eines Polyethylenglykols umfassend 2 bis 5 Oxyethylen-Einheiten, einen zweiten Ether eines Stearylalkohols und eines Polyethylenglykols umfassend 15 bis 25 Oxyethylen-Einheiten, und ein Copolymer von Acrylamid-2-methylpropansulfonat umfasst.

2. Produkt nach Anspruch 1, **dadurch gekennzeichnet, dass** die Öle hydriertes Methylrosinat und hydriertes Polycylcopentadien umfassen.

3. Produkt nach Anspruch 2, **dadurch gekennzeichnet, dass** das hydrierte Methylrosinat zwischen 10 und 15 Massen-% der Masse des Produkts darstellt.

4. Produkt nach einem der Ansprüche 2 und 3, **dadurch gekennzeichnet, dass** das hydrierte Polycyclopentadien zwischen 2 und 5 Massen-% der Masse des Produkts darstellt.

5. Produkt nach einem der vorhergehenden Ansprüche, **dadurch gekennzeichnet, dass** der erste Ether die Bezeichnung INCI STEARETH-2 trägt und der zweite Ether die Bezeichnung INCI STEARETH-21 trägt.

6. Produkt nach einem der vorhergehenden Ansprüche, **dadurch gekennzeichnet, dass** das Copolymer von Acrylamido-2-methylpropansulfonat und Hydroxyethylacrylat zwischen 0,2 und 1,0 Massen-% der Masse des Produkts darstellt.

7. Verfahren zum Schminken der Lippen, das darin besteht, ein Produkt nach einem der Ansprüche 1 bis 6 auf die Lippen aufzutragen.

8. Fläschchen, das mit einem Applikator und einer Kappe versehen ist, wobei das Fläschchen transparent ist und ein Produkt nach einem der Ansprüche 1 bis 6 enthält, wobei der Applikator ein Pinsel oder ein Schaumstoff ist.

## Claims

1. An oil-in-water emulsion liquid product for making up and caring for the lips comprising:
- from 25% to 45% of a mixture comprising water and at least one polyol,
- from 35% to 60% by weight of a mixture of oils, each oil having a refractive index, measured at a temperature ranging from 20°C to 25°C, which is greater than or equal to 1.460,
- from 0.01% to 20% by weight of a coloring material,
the percentages being expressed with respect to the weight of the emulsion,
**characterized in that** it comprises a first ether of stearyl alcohol and of a polyethylene glycol comprising from 2 to 5 oxyethylene units, a second ether of stearyl alcohol and of a polyethylene glycol comprising from 15 to 25 oxyethylene units, and an acrylamido-2-methylpropanesulfonate copolymer.

2. The product as claimed in claim 1, **characterized in that** the oils comprise methyl hydrogenated rosinate and hydrogenated polycyclopentadiene.

3. The product as claimed in claim 2, **characterized in that** the methyl hydrogenated rosinate represents between 10% and 15% by weight of the weight of the product.

4. The product as claimed in either of claims 2 and 3, **characterized in that** the hydrogenated polycyclopentadiene represents between 2% and 5% by weight of the weight of the product.

5. The product as claimed in one of the preceding claims, **characterized in that** the first ether has the INCI name STEARETH-2 and the second ether has the INCI name STEARETH-21.

6. The product as claimed in one of the preceding claims, **characterized in that** the copolymer of acrylamido-2-methylpropanesulfonate and of hydroxyethyl acrylate represents between 0.2% and 1.0% by weight of the weight of the product.

7. A process for making up the lips, which consists in applying, to the lips, a product as claimed in one of claims 1 to 6.

8. A bottle having an application means and a cap, which bottle is transparent and contains a product as claimed in one of claims 1 to 6, said application means being a fine brush or a cellular foam.
